(19) Europäisches Patentamt
European Patent Office
Office européen des brevets

(11) EP 3 284 399 A1

(12) EUROPEAN PATENT APPLICATION
published in accordance with Art. 153(4) EPC

(43) Date of publication:
21.02.2018 Bulletin 2018/08

(51) Int Cl.:
*A61B 5/00* (2006.01)

(21) Application number: 16830091.1

(86) International application number:
PCT/JP2016/057832

(22) Date of filing: 11.03.2016

(87) International publication number:
WO 2017/017986 (02.02.2017 Gazette 2017/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 24.07.2015 JP 2015147184

(71) Applicant: Medcare, Inc.
Tokyo 163-1030 (JP)

(72) Inventor: AKASHI, Hideyuki
Shinjuku-ku, Tokyo 163-1030 (JP)

(74) Representative: Weickmann & Weickmann
PartmbB
Postfach 860 820
81635 München (DE)

(54) **BIOLOGICAL MONITORING DEVICE AND BIOLOGICAL MONITORING PROGRAM**

(57) A biological monitoring device for accurately detecting a sudden abnormality of a living organism is provided. A biological information measurement unit (33) measures biological information of patient P for each predetermined measurement cycle by vital sensors (10)(11), and stores in a memory (32). A dispersion degree calculation unit (34) calculates dispersion degree of the biological information in a predetermined measurement period Tw, referring to time series data of the biological information stored in the memory (32). A divergence degree calculation unit (35) calculates, for evaluation biological information which is the biological information measured at a predetermined point after measurement period Tw, a divergence degree of the evaluation biological information from a dispersion range of the biological information in measurement period Tw, based on the dispersion degree. An abnormality determination unit (36) determines that patient P is in abnormal state, when the divergence degree is a predetermined level or more.

FIG.1

## Description

Technical Field

The present invention relates to a biological monitoring device for measuring biological information and determining the health state of a living organism.

Background Art

A system of measuring, for example by a wearable sensor worn by a subject (living organism), biological information (vital data) such as the body temperature, pulse, and movement of the subject and calculating the disturbance degree of the biological rhythm of the subject has been conventionally proposed (for example, see Patent Literature 1).

In the system described in Patent Literature 1, regarding time series data (physiological index time series data) of biological information measured between predetermined times, the disturbance degree of the biological rhythm at inspection time is determined based on the amount of deviation in the physiological index time series data between normal time and inspection time.

Citation List

Patent Literature

Patent Literature 1: Japanese Patent Application Laid-Open No. 2012-239799

Summary of Invention

Technical Problem

The inventors of the present application have learned that, in the case of using the disturbance degree of the biological rhythm based on the amount of deviation in the physiological index time series data between normal time and inspection time as in the system described in Patent Literature 1, it is sometimes difficult to accurately detect a sudden abnormality of the living organism.

The present invention has been made in view of the above, and has an object of providing a biological monitoring device and a biological monitoring program that can accurately detect a sudden abnormality of a living organism.

Solution to Problem

A biological monitoring device according to the present invention includes: a biological information measurement unit that measures biological information of a monitoring target for each predetermined cycle; a biological information holding unit that holds time series data of the biological information measured by the biological information measurement unit; a dispersion degree calculation unit that calculates a dispersion degree of the biological information in a predetermined measurement period, with reference to the time series data held in the biological information holding unit; a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and an abnormality determination unit that determines that the monitoring target is in an abnormal state, in a case where the divergence degree is greater than or equal to a predetermined level.

According to the present invention, the biological information measurement unit measures the biological information of the monitoring target for each predetermined cycle, and the biological information holding unit holds the time series data of the biological information. The dispersion degree calculation unit then calculates the dispersion degree of the biological information in the measurement period. The divergence degree calculation unit calculates, for the evaluation biological information measured at the predetermined time point after the measurement period, the divergence degree from the dispersion range of the biological information in the measurement period, based on the dispersion degree.

Here, the dispersion degree indicates the tendency (trend) of change of the biological state in the past measurement period, so that the divergence degree indicates the degree of deviation of the biological information from the past tendency. Hence, the abnormality determination unit can accurately determine that a living organism which is the monitoring target has a sudden abnormality, in the case where the divergence degree is greater than or equal to the predetermined level.

Moreover, the dispersion degree calculation unit calculates a standard deviation of the biological information in the measurement period, as the dispersion degree, and the divergence degree calculation unit calculates, as the divergence degree, a value obtained by replacing the evaluation biological information with a deviation value of the biological information in the measurement period, using the standard deviation and an average value of the biological information in the measurement period.

With this structure, the dispersion degree calculation unit calculates the standard deviation of the biological information in the measurement period, so that the divergence degree calculation unit can easily calculate the divergence degree using the standard deviation.

Moreover, the predetermined time point is a measurement point in time of the biological information in a most recent predetermined cycle, and the measurement period is a period of past several cycles from a predetermined cycle immediately preceding the most recent predetermined cycle.

With this structure, by determining, for the biological information in the most recent predetermined cycle, the degree of deviation from the tendency of change of the biological information in the immediately previous measurement period, an abnormality of the living organism which is the monitoring target can be determined in real time.

Moreover, the biological information measurement unit measures a plurality of types of biological information, the biological information holding unit holds time series data of each of the plurality of types of biological information measured by the biological information measurement unit, the dispersion degree calculation unit individually calculates a dispersion degree of each of the plurality of types of biological information in the measurement period, with reference to the time series data of the plurality of types of biological information held in the biological information holding unit, and the divergence degree calculation unit: calculates, for the evaluation biological information of each of the plurality of types at the predetermined time point, a divergence degree of the evaluation biological information from a dispersion tendency of the biological information in the measurement period, based on the dispersion degree; and calculates a weighted average of the calculated divergence degrees, as the divergence degree that is compared with the predetermined level by the abnormality determination unit.

With this structure, the biological information measurement unit measures the plurality of types of biological information, and the dispersion degree calculation unit calculates the dispersion degree for each of the plurality of types of biological information. The divergence degree calculation unit then calculates the weighted average of the divergence degrees of the plurality of types of biological information, as the divergence degree that is compared with the predetermined level by the abnormality determination unit. In this case, since the status of deviation from the past change tendency for the plurality of types of biological information can be determined based on one parameter (the weighted average of the divergence degrees of the plurality of types of biological information), a sudden abnormal state in a wider range can be determined easily.

Moreover, the biological information measurement unit measures a heart rate of the monitoring target, as the biological information, and the abnormality determination unit determines that the monitoring target is in the abnormal state due to paroxysmal atrial fibrillation, in the case where the divergence degree is greater than or equal to the predetermined level.

With this structure, in the case where the divergence degree is greater than or equal to the predetermined level, an abnormal state due to paroxysmal atrial fibrillation can be determined in recognition that the heart rate of the living organism as the monitoring target changes from a normal rhythm to a rhythm of atrial fibrillation.

Moreover, the biological information measurement unit measures a respiration rate of the monitoring target, as the biological information, and the abnormality determination unit determines that the monitoring target is in the abnormal state due to Cheyne-Stokes respiration, in the case where the divergence degree is greater than or equal to the predetermined level.

With this structure, in the case where the divergence degree is greater than or equal to the predetermined level, an abnormal state due to Cheyne-Stokes respiration can be determined in recognition that the respiration status of the living organism as the monitoring target cyclically switches between hypoventilation and hyperventilation.

Moreover, the biological information measurement unit measures a respiration rate and a posture of the monitoring target, as the biological information, and the abnormality determination unit determines that the monitoring target is in the abnormal state due to orthopnea, in the case where the divergence degree relating to the respiration rate is greater than or equal to the predetermined level and a posture change of the monitoring target is detected from measurement data of the posture.

With this structure, in the case where the divergence degree is greater than or equal to the predetermined level and the posture change of the monitoring target is detected from the measurement data of the posture, an abnormal state due to orthopnea can be determined in recognition that the respiration rate of the monitoring target deviates from the past change tendency with the posture change of the monitoring target.

Moreover, the monitoring target is a living organism lying on a bed, the biological information measurement unit measures a movement of the monitoring target, as the biological information, and the abnormality determination unit determines that the monitoring target is in the abnormal state of having a high possibility of falling off the bed, in the case where the divergence degree is greater than or equal to the predetermined level.

With this structure, in the case where the divergence degree is greater than or equal to the predetermined level, an abnormal state of having a high possibility of falling off the bed can be determined in recognition that the movement of the living organism on the bed changes suddenly.

A biological monitoring program according to the present invention causes a computer to function as: a biological information measurement unit that measures biological information of a monitoring target for each predetermined cycle; a biological information holding unit that holds time series data of the biological information measured by the biological information measurement unit; a dispersion degree calculation unit that calculates a dispersion degree of the biological information in a predetermined measurement period, with reference to the time series data held in the biological information holding unit; a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and an abnormality determination unit that determines that the monitoring target is in an abnormal state, in a case where the divergence degree is greater than or equal to a predetermined level.

By executing the biological monitoring program according to the present invention by a computer, the structure of the biological monitoring device described above can be realized.

Brief Description of Drawings

FIG. 1 is a diagram of a biological monitoring device.
FIG. 2 is a diagram of a nurse call handset.
FIG. 3A is a diagram of a screen in normal time when no nurse call is made.
FIG. 3B is a diagram of a screen when a nurse call is made.
FIG. 4 is a diagram of a staff mobile terminal.
FIG. 5 is a first flowchart of a living organism abnormality determination process.
FIG. 6 is a second flowchart of the living organism abnormality determination process.
FIG. 7 is an explanatory diagram of relative thresholds and absolute thresholds in the abnormality determination process.

Description of Embodiments

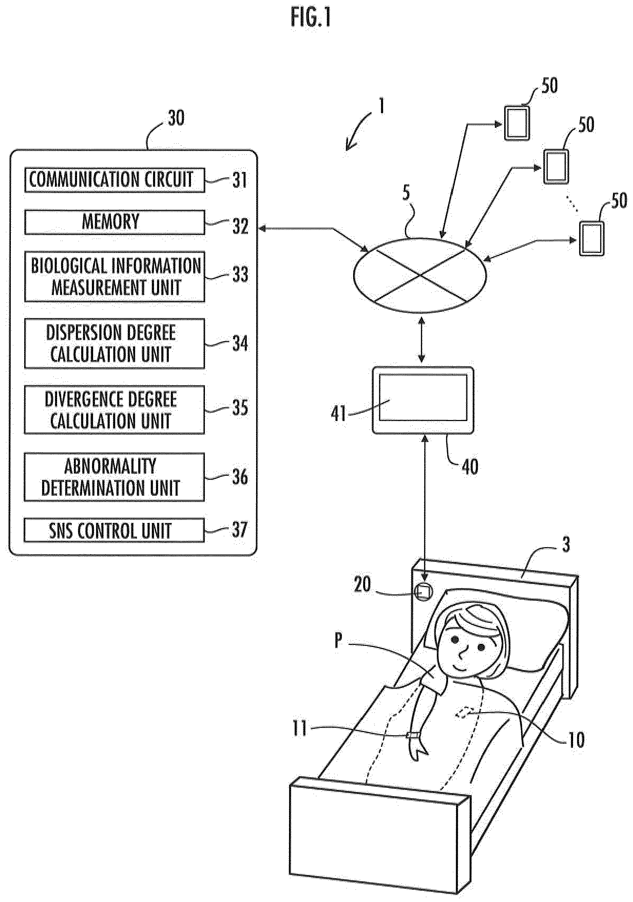

An embodiment of the present invention is described below, with reference to FIGS. 1 to 7. As illustrated in FIG. 1, a biological monitoring device 30 in this embodiment constitutes a nurse call system 1 with a nurse call handset 20, a nurse call base unit 40, and a mobile terminal 50 carried by each nurse.

The nurse call system 1 is installed in a hospital, and has a structure for a nurse or a doctor responding to a call from a hospitalized patient. The biological monitoring device 30 performs data communication between the nurse call base unit 40 and the mobile terminal 50 carried by each nurse, via a network 5.

The nurse call handset 20 is attached to the headboard of a bed 3, and has a function of performing wireless (e.g. Bluetooth®) data communication with vital sensors 10 and 11 worn by a patient P (corresponding to a living organism as the monitoring target according to the present invention) lying on the bed 3, and a function of performing wired or wireless data communication with the biological monitoring device 30 via the nurse call base unit 40.

In this embodiment, the chest-worn vital sensor 10 worn on the chest of the patient P and the wristband vital sensor 11 worn around the wrist of the patient P are used as vital sensors. A contactless vital sensor for measuring the body temperature, movement, pulse, respiration state, etc. of the patient P contactlessly may be used.

The chest-worn vital sensor 10 measures biological information (vital data) such as the electrocardiogram, heart rate, respiration state, posture, and body surface temperature of the patient P, and transmits the measurement data to the nurse call handset 20. The wristband vital sensor 11 measures biological information such as the pulse and activity amount of the patient P, and transmits the measurement data to the nurse call handset 20.

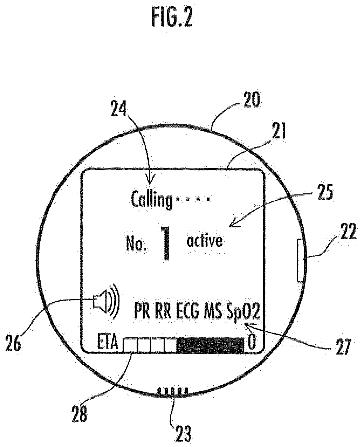

As illustrated in FIG. 2, the nurse call handset 20 includes a touch panel 21, a communication terminal 22, and a speaker/microphone 23. The patient P can call a nurse by touching the touch panel 21, and talk with the nurse via the speaker/microphone 23.

The touch panel 21 displays a calling display 24 indicating that a call to a nurse is being made, an operating state display 25 indicating the number and operating state of the handset 20, a talking display 26 indicating that talking is in progress, a sensor status display 27 indicating the reception status of the measurement data of biological information from the vital sensors 10 and 11, and an ETA display 28 indicating the estimated time of arrival (ETA) of the nurse.

The nurse call handset 20 performs wireless or wired data communication with the nurse call base unit 40. The nurse call handset 20 transmits the measurement data of biological information received from the vital sensors 10 and 11, to the nurse call base unit 40. The nurse call handset 20 also performs transmission and reception of talk data with the nurse call base unit 40.

The biological monitoring device 30 is composed of a computer including a communication circuit 31, a memory 32 (including the function of the biological information holding unit according to the present invention), a CPU (not illustrated), various interface circuits, and the like. The CPU executes a control program (biological monitoring program) of the biological monitoring device 30 held in the memory 32, to function as a biological information measurement unit 33, a dispersion degree calculation unit 34, a divergence degree calculation unit 35, an abnormality determination unit 36, and an SNS control unit 37.

The biological information measurement unit 33 receives the measurement data of biological information from the vital sensors 10 and 11, via the nurse call handset 20 and the nurse call base unit 40. The biological information measurement unit 33 calculates an average value of the biological information for each predetermined cycle $\Delta t$ (e.g. 1 second), sets the calculated average value as biological information in each $\Delta t$, and stores time series data of the biological information in the memory 32.

The dispersion degree calculation unit 34 calculates a dispersion (variability, irregularity) degree of the biological information in a measurement period Tw (a period corresponding to several cycles Δt), based on the measurement value of the biological information in each Δt calculated by the biological information measurement unit 33. The divergence degree calculation unit 35 calculates a divergence degree indicating how much the biological information in the most recent Δt (corresponding to the evaluation biological information at the predetermined time point according to the present invention) calculated by the biological information measurement unit 33 diverges from the dispersion tendency of the biological information in the past measurement period Tw calculated by the dispersion degree calculation unit 34.

The abnormality determination unit 36 determines whether or not the patient P has an abnormality, based on the divergence degree calculated by the divergence degree calculation unit 35. In the case of determining that the patient P has an abnormality, the abnormality determination unit 36 transmits "abnormality notification data" indicating that the patient P has an abnormality, to the nurse call base unit 40 and the mobile terminal 50. The processes by the biological information measurement unit 33, the dispersion degree calculation unit 34, the divergence degree calculation unit 35, and the abnormality determination unit 36 will be described in detail later.

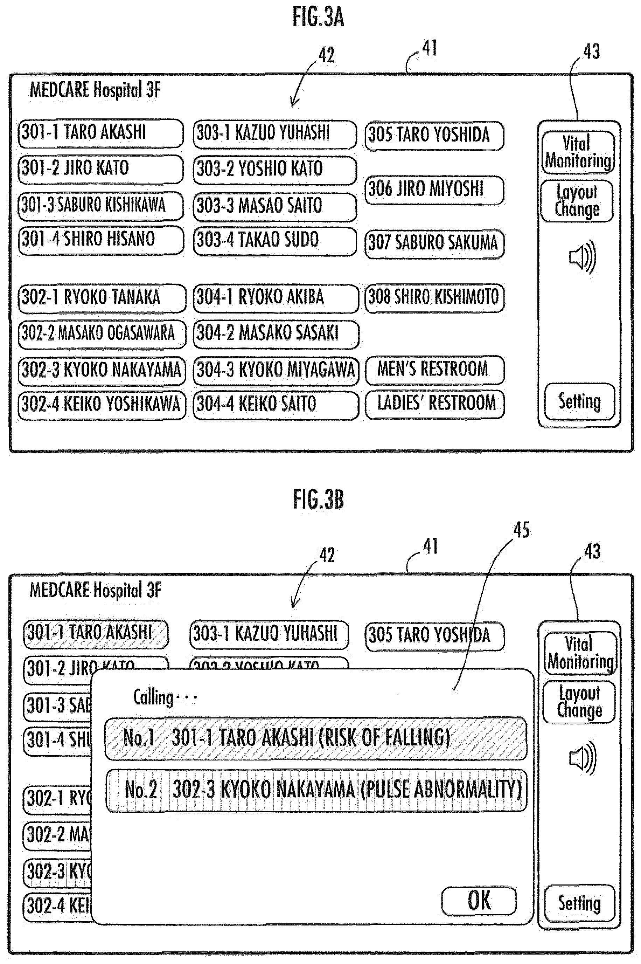

The nurse call base unit 40 includes a touch panel 41. As illustrated in FIG. 3A, the nurse call base unit 40 displays, on the touch panel 41, a list 42 of whether or not there is a call from the nurse call handset 20 installed at each bed in each hospital room and each common utility (restroom in FIG. 3A), and an operation part 43 for operating the nurse call base unit 40.

Upon receiving "abnormality notification data" from the biological monitoring device 30, the nurse call base unit 40 displays a pop-up display 45 notifying the hospital room and name of a patient having an abnormality on the touch panel 41, as illustrated in FIG. 3B. In FIG. 3B, "risk of falling" is displayed for the patient (Taro Akashi) in bed 1 in room 301, and "pulse abnormality" is displayed for the patient (Kyoko Nakayama) in bed 3 in room 302.

When the patient P touches the nurse call handset 20 to make a call, the biological monitoring device 30 highlights the name of the patient who has touched the nurse call handset 20, without producing the pop-up display 45 illustrated in FIG. 3B.

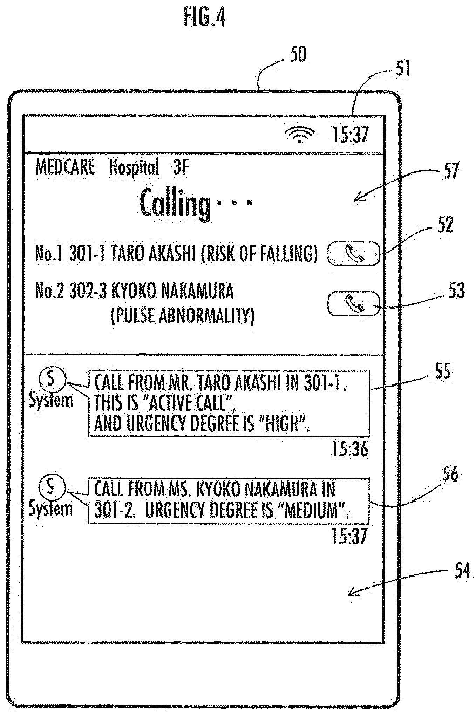

The mobile terminal 50, upon receiving "abnormality notification data" from the biological monitoring device 30, displays a call display 57 indicating the status of the call from the patient, response buttons 52 and 53, and a communication screen 54 on a touch panel 51, as illustrated in FIG. 4.

The SNS control unit 37 in the biological monitoring device 30 provides an SNS (Social Networking Service)-like information sharing service to each nurse carrying the mobile terminal 50. The SNS control unit 37 provides an application for an information sharing service to each mobile terminal 50. By each mobile terminal 50 executing this application, information sharing between the mobile terminals 50 is enabled.

The nurses carrying the mobile terminals 50 share information about each patient having an abnormality, using the information sharing service provided by the SNS control unit 37. In detail, the SNS control unit 37 has abnormal state details 55 and 56 displayed on the communication screen 54 of each mobile terminal 50, and receives information about patient treatment and the like input by a nurse. The SNS control unit 37 distributes the input information to each mobile terminal 50.

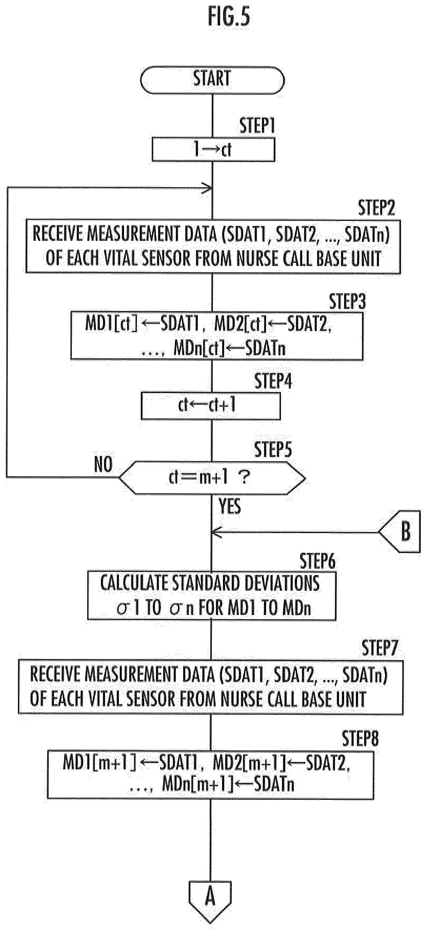

The processes by the dispersion degree calculation unit 34, the divergence degree calculation unit 35, and the abnormality determination unit 36 are described below, with reference to flowcharts illustrated in FIGS. 5 to 6.

STEP 1 to STEP 6 in FIG. 5 are the process by the dispersion degree calculation unit 34. The dispersion degree calculation unit 34 prepares n array variables MD1 to MDn each having (m + 1) elements and a counter variable ct, in order to hold measurement data of n pieces (n types) of biological information measured (m + 1) times by the vital sensors 10 and 11.

The dispersion degree calculation unit 34 sets the counter variable ct to 1 in STEP 1, and repeatedly performs a process of a loop of STEP 2 to STEP 5 until the counter variable ct reaches m + 1 in STEP 5.

In STEP 2, the dispersion degree calculation unit 34 receives measurement data SDAT1, SDAT2, ..., SDATn of n pieces (n types) of biological information from the vital sensors 10 and 11. In the next STEP 3, the dispersion degree calculation unit 34 writes the received measurement data SDAT1, SDAT2, ..., SDATn respectively to the array variables MD1[ct], MD2[ct], ..., MDn[ct]. In the next STEP 4, the dispersion degree calculation unit 34 increments the counter variable ct. The dispersion degree calculation unit 34 then advances to STEP 5.

In the case where the counter variable ct is m + 1 in STEP 5, i.e. in the case where measurement data SDAT1, SDAT2, ..., SDATn corresponding to m times measurements are written in the array elements 1 to m of the array variables MD1 to MDn, the dispersion degree calculation unit 34 advances to STEP 6. Regarding the measurement data written in the array elements 1 to m of the array variables MD1 to MDn, the dispersion degree calculation unit 34 calculates standard deviations $\sigma 1$ to $\sigma n$ for the respective types of biological information. For example, regarding the array variable MD1, the dispersion degree calculation unit 34 calculates the standard deviation $\sigma 1$ for the array variables MD1[1] to MD1[m], according to the following Expressions (1) and (2).

[Math. 1]

[Math. 1]

$$\sigma 1 = \frac{1}{m}\sum_{i=1}^{m} MD1[i] \quad ……(1)$$

[Math. 2]

$$\sigma 1^2 = \frac{1}{m}\sum_{i=1}^{m}\{MD1[i]-\sigma 1\}^2 \quad ……(2)$$

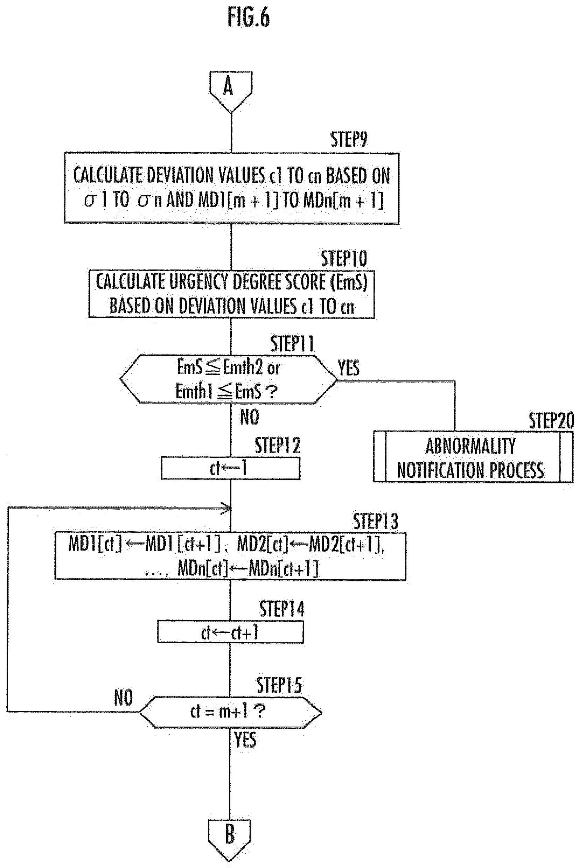

The next STEP 7 to STEP 10 in FIG. 6 are the processes by the divergence degree calculation unit 35. In STEP 7, the divergence degree calculation unit 35 receives measurement data SDAT1, SDAT2, …., SDATn of the vital sensors 10 and 11, from the nurse call base unit 40. In the next STEP 8, the divergence degree calculation unit 35 writes the received measurement data SDAT1, SDAT2, …., SDATn to the respective array elements m + 1 of the array variables MD1 to MDn.

In the next STEP 9 in FIG. 6, the divergence degree calculation unit 35 calculates a deviation value σ1 by replacing the most recent measurement data MD1[m + 1] based on the standard deviation σ1 of the measurement data MD1[1] to MD1[m] in the immediately previous measurement period Tw, according to the following Expression (3).

[Math. 3]

$$\sigma 1 = \frac{MD1[m+1]-\sigma 1}{\sigma 1}\times 10 + 50 \quad ……(3)$$

For the array variables MD2 to MDn, the divergence degree calculation unit 35 similarly calculates deviation values σ2 to σn of MD2[m+1] to MDn[m+1].

In the next STEP 10, the divergence degree calculation unit 35 calculates a weighted average of the deviation values σ1 to σn as an urgency degree score EmS, according to the following Expression (4).

[Math. 4]

$$EmS = \frac{1}{n}(k1 \cdot \sigma 1 + k2 \cdot \sigma 2 + …… + kn \cdot \sigma n) \quad ……(4)$$

Here, k1, k2, …., kn are coefficients of the weighted average, and are determined by calculating, from a vector autoregressive model (VAR model), a VAR(p) model in the following Expression (5) and performing tuning from an impulse reaction function.

[Math. 5]

$$y_i = c + \Phi_1 y_{i-1} + …… + u_i \quad ……(5)$$

The next STEP 11 is the process by the abnormality determination unit 36. In the case where the urgency degree score EmS is greater than or equal to an upper-limit threshold Emth1 or less than or equal to a lower-limit threshold Emth2 set based on the standard deviations σ1 to σn in the immediately previous measurement period Tw, the abnormality determination unit 36 determines that the patient P has a sudden abnormality, and advances to STEP 20. In STEP 20, the abnormality determination unit 36 transmits "abnormality notification data" to the nurse call base unit 40 and the mobile terminal 50 carried by each nurse, via the network 5 (abnormality notification process). The upper-limit threshold Emth1 and the lower-limit threshold Emth2 are set based on the standard deviations σ1 to σn, past measurement results, and the like.

As a result, the nurse call base unit 40 produces the pop-up display illustrated in FIG. 3B, as mentioned above. In addition, the mobile terminal 50 carried by each nurse displays the abnormality notification screen illustrated in FIG. 4, as mentioned above.

In the case where the urgency degree score EmS is greater than the lower-limit threshold Emth2 and less than the upper-limit threshold Emth1 in STEP 11, the abnormality determination unit 36 determines that the patient P has no abnormality, and advances to STEP 12.

STEP 12 to STEP 15 are the processes by the dispersion degree calculation unit 34. The dispersion degree calculation unit 34 sets the counter variable ct to 1 in STEP 12, and shifts the measurement data held in the array variables MD1 to MDn to the array elements 2 to m + 1 of the array variables MD1 to MDn by a process a by m to the array elements 1 to MDn of STEP 13 to STEP 15.

For example, regarding the array variable MD1, the dispersion degree calculation unit 34 shifts the measurement data held in MD1[2] to MD1[m + 1] respectively to MD1[1] to MD1[m]. The same applies to the array variables MD2 to MDm.

In the case where the counter variable ct is m + 1 in STEP 15, the dispersion degree calculation unit 34 advances to STEP 6 in FIG. 5. The dispersion degree calculation unit 34 calculates the standard deviations σ1 to σn respectively for the array variables MD1 to MDn with the shifted measurement data. Thus, the standard deviations σ1 to σn are updated with the values based on the measurement data in the immediately previous measurement period Tw.

Subsequently, each time measurement data SDAT1 to SDATn of the vital sensors 10 and 11 are received from the nurse call base unit 40 in STEP 7, the divergence degree calculation unit 35 calculates the urgency degree score EmS in STEP 8 to STEP 10, and the abnormality determination unit 36 determines in real time whether or not the patient P has an abnormality in STEP 11. The dispersion degree calculation unit 34 then updates the standard deviations σ1 to σn based on the immediately previous meas-

urement period Tw in STEP 12 to STEP 15 and STEP 6.

[Other embodiments]

Although the urgency degree score EmS is calculated by taking a weighted average of a plurality of types of biological information according to Expression (4) in the foregoing embodiment, the urgency degree score EmS may be calculated for each type of biological information to determine whether or not the patient P has an abnormality.

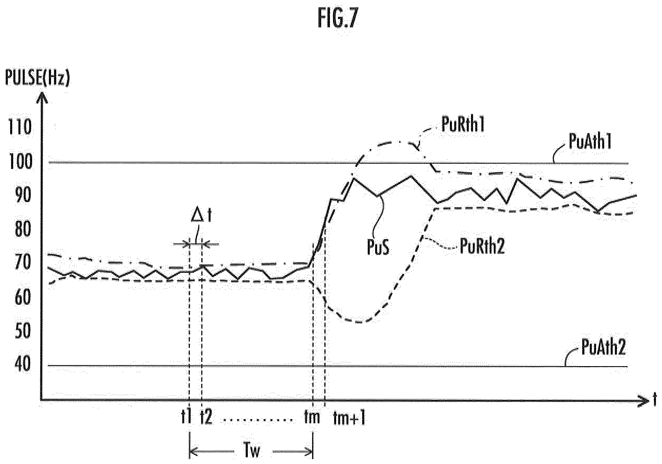

FIG. 7 illustrates an example of performing the processes by the dispersion degree calculation unit 34, the divergence degree calculation unit 35, and the abnormality determination unit 36 with regard to pulse. The vertical axis is set to pulse (Hz), and the horizontal axis is set to time (t).

In FIG. 7, PuS denotes a measurement value of pulse by the vital sensor 11, PuRth1 denotes a relative upper limit, PuRth2 denotes a relative lower limit, PuAth1 denotes an absolute upper limit, and PuAth2 denotes an absolute lower limit.

The dispersion degree calculation unit 34 receives measurement data of the vital sensor 11 from the nurse call base unit 40 at t1, t2, ..., tm, and tm + 1 for each predetermined cycle $\Delta t$. At tm at which m pieces of measurement data have been received, the dispersion degree calculation unit 34 calculates a standard deviation $\sigma p$ of the measurement value of the pulse in a measurement period Tw (corresponding to the process in STEP 1 to STEP 6 in FIG. 5).

The divergence degree calculation unit 35 sets ap + $2\sigma p$ as the relative upper limit PuRth1, and sets ap - $2\sigma p$ as the relative lower limit PuRth2, where ap is an average value of the measurement value of the pulse in the measurement period Tw. In this case, if the measurement value of the pulse at tm is within the range of the relative lower limit PuRth2 to the relative upper limit PuRth1, the divergence degree of the measurement value of the pulse at tm with respect to the dispersion tendency of the pulse in the measurement period Tw is determined to be a normal level (less than the predetermined level according to the present invention).

If the measurement value of the pulse at tm is outside the range of the relative lower limit PuRth2 to the relative upper limit PuRth1, the abnormality determination unit 36 determines that the patient P is in an abnormal state.

[Determination for specific cases]

(1) Paroxysmal atrial fibrillation

An abnormal state due to paroxysmal atrial fibrillation can be determined by performing the processes by the dispersion degree calculation unit 34, the divergence degree calculation unit 35, and the abnormality determination unit 36 for measurement data of the heart rate of the patient P by the vital sensor 10 to calculate the divergence degree of the most recent heart rate with respect to the dispersion degree of the heart rate in the past measurement period Tw.

(2) Cheyne-Stokes respiration

An abnormal state due to Cheyne-Stokes respiration can be determined by performing the processes by the dispersion degree calculation unit 34, the divergence degree calculation unit 35, and the abnormality determination unit 36 for measurement data of the respiration rate of the patient P by the vital sensor 10 to calculate the divergence degree of the most recent respiration rate with respect to the dispersion degree of the respiration rate in the past measurement period Tw.

(3) Orthopnea

An abnormal state due to orthopnea can be determined as follows: The processes by the dispersion degree calculation unit 34, the divergence degree calculation unit 35, and the abnormality determination unit 36 are performed for measurement data of the respiration rate of the patient P by the vital sensor 10, to calculate the divergence degree of the most recent respiration rate with respect to the dispersion degree of the respiration rate in the past measurement period Tw. In the case where the divergence degree is greater than or equal to a predetermined level and the vital sensor 10 detects a posture change of the patient P, the patient P is determined to be in an abnormal state due to orthopnea.

(4) Falling off a bed

An abnormal state of the patient P having a high possibility of falling off the bed can be determined by performing the processes by the dispersion degree calculation unit 34, the divergence degree calculation unit 35, and the abnormality determination unit 36 based on measurement data of the posture of the patient P by the vital sensor 10 to calculate the divergence degree of the most recent posture with respect to the dispersion degree of the posture of the patient P in the past measurement period Tw.

[Applications of the present invention]

Although the foregoing embodiment describes an example where the biological monitoring device according to the present invention is part of a nurse call system installed in a hospital, the biological monitoring device according to the present invention can also be used in the case of monitoring biological information of nursing home residents, home care patients, etc.

The present invention is also applicable to living organisms (e.g. dogs, cats, or the like) other than humans.

[Modifications]

Although the biological monitoring device according to the present invention has a server function and receives the measurement data of the vital sensors via the network in the foregoing embodiment, the biological monitoring device according to the present invention may receive the measurement data of the vital sensors directly without involving the network.

Although a standard deviation is used as the dispersion degree according to the present invention in the foregoing embodiment, other indexes such as a measurement value change range (maximum measurement value - minimum measurement value) may be used.

Description of Reference Numerals

1　　　nurse call system
10　　(chest-worn) vital sensor
11　　(wristband) vital sensor,
20　　nurse call handset
30　　biological monitoring device
33　　biological information measurement unit
34　　dispersion degree calculation unit
35　　divergence degree calculation unit
36　　abnormality determination unit
37　　SNS control unit
40　　nurse call base unit
50　　mobile terminal (carried by nurse)

**Claims**

1. A biological monitoring device comprising:

   a biological information measurement unit that measures biological information of a monitoring target for each predetermined cycle;
   a biological information holding unit that holds time series data of the biological information measured by the biological information measurement unit;
   a dispersion degree calculation unit that calculates a dispersion degree of the biological information in a predetermined measurement period, with reference to the time series data held in the biological information holding unit;
   a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and
   an abnormality determination unit that determines that the monitoring target is in an abnormal state, in a case where the divergence degree is greater than or equal to a predetermined level.

2. The biological monitoring device according to claim 1, wherein the dispersion degree calculation unit calculates a standard deviation of the biological information in the measurement period, as the dispersion degree, and
   wherein the divergence degree calculation unit calculates, as the divergence degree, a value obtained by replacing the evaluation biological information with a deviation value of the biological information in the measurement period, using the standard deviation and an average value of the biological information in the measurement period.

3. The biological monitoring device according to claim 1, wherein the predetermined time point is a measurement point in time of the biological information in a most recent predetermined cycle, and the measurement period is a period of past several cycles from a predetermined cycle immediately preceding the most recent predetermined cycle.

4. The biological monitoring device according to claim 1, wherein the biological information measurement unit measures a plurality of types of biological information,
   wherein the biological information holding unit holds time series data of the plurality of types of biological information measured by the biological information measurement unit,
   wherein the dispersion degree calculation unit individually calculates a dispersion degree of each of the plurality of types of biological information in the measurement period, with reference to the time series data of the plurality of types of biological information held in the biological information holding unit, and
   wherein the divergence degree calculation unit: calculates, for the evaluation biological information of each of the plurality of types at the predetermined time point, a divergence degree of the evaluation biological information from a dispersion tendency of the biological information in the measurement period, based on the dispersion degree; and calculates a weighted average of the calculated divergence degrees, as the divergence degree that is compared with the predetermined level by the abnormality determination unit.

5. The biological monitoring device according to claim 1, wherein the biological information measurement unit measures a heart rate of the monitoring target, as the biological information, and
wherein the abnormality determination unit determines that the monitoring target is in the abnormal state due to paroxysmal atrial fibrillation, in the case where the divergence degree is greater than or equal to the predetermined level.

6. The biological monitoring device according to claim 1, wherein the biological information measurement unit measures a respiration rate of the monitoring target, as the biological information, and
wherein the abnormality determination unit determines that the monitoring target is in the abnormal state due to Cheyne-Stokes respiration, in the case where the divergence degree is greater than or equal to the predetermined level.

7. The biological monitoring device according to claim 1, wherein the biological information measurement unit measures a respiration rate and a posture of the monitoring target, as the biological information, and
wherein the abnormality determination unit determines that the monitoring target is in the abnormal state due to orthopnea, in the case where the divergence degree relating to the respiration rate is greater than or equal to the predetermined level and a posture change of the monitoring target is detected from measurement data of the posture.

8. The biological monitoring device according to claim 1, wherein the monitoring target is a living organism lying on a bed,
wherein the biological information measurement unit measures a movement of the monitoring target, as the biological information, and
wherein the abnormality determination unit determines that the monitoring target is in the abnormal state of having a high possibility of falling off the bed, in the case where the divergence degree is greater than or equal to the predetermined level.

9. A biological monitoring program causing a computer to function as:

 a biological information measurement unit that measures biological information of a monitoring target for each predetermined cycle;
 a biological information holding unit that holds time series data of the biological information measured by the biological information measurement unit;
 a dispersion degree calculation unit that calculates a dispersion degree of the biological information in a predetermined measurement period, with reference to the time series data held in the biological information holding unit;
 a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and
 an abnormality determination unit that determines that the monitoring target is in an abnormal state, in a case where the divergence degree is greater than or equal to a predetermined level.

**Amended claims under Art. 19.1 PCT**

1. A biological monitoring device comprising:

 a biological information measurement unit that measures biological information of a monitoring target for each predetermined cycle;
 a biological information holding unit that holds time series data of the biological information measured by the biological information measurement unit;
 a dispersion degree calculation unit that calculates a dispersion degree of the biological information in a predetermined measurement period, with reference to the time series data held in the biological information holding unit;
 a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and
 an abnormality determination unit that determines that the monitoring target is in an abnormal state, in a case where the divergence degree is greater than or equal to a predetermined level,
 wherein the biological information measurement unit measures a plurality of types of biological information,
 wherein the biological information holding unit holds time series data of the plurality of types of biological information measured by the biological information measurement unit,
 wherein the dispersion degree calculation unit individually calculates a dispersion degree of each of the plurality of types of biological information in the measurement period, with reference to the time series data of the plurality of types of biological information held in the biological information holding unit, and

wherein the divergence degree calculation unit: calculates, for the evaluation biological information of each of the plurality of types at the predetermined time point, a divergence degree of the evaluation biological information from a dispersion tendency of the biological information in the measurement period, based on the dispersion degree; and calculates a weighted average of the calculated divergence degrees, as the divergence degree that is compared with the predetermined level by the abnormality determination unit.

2. The biological monitoring device according to claim 1, wherein the dispersion degree calculation unit calculates a standard deviation of the biological information in the measurement period, as the dispersion degree, and

wherein the divergence degree calculation unit calculates, as the divergence degree, a value obtained by replacing the evaluation biological information with a deviation value of the biological information in the measurement period, using the standard deviation and an average value of the biological information in the measurement period.

3. The biological monitoring device according to claim 1, wherein the predetermined time point is a measurement point in time of the biological information in a most recent predetermined cycle, and the measurement period is a period of past several cycles from a predetermined cycle immediately preceding the most recent predetermined cycle.

4. (Canceled).

5. A biological monitoring device comprising:

a biological information measurement unit that measures biological information of a monitoring target for each predetermined cycle;
a biological information holding unit that holds time series data of the biological information measured by the biological information measurement unit;
a dispersion degree calculation unit that calculates a dispersion degree of the biological information in a predetermined measurement period, with reference to the time series data held in the biological information holding unit;
a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and
an abnormality determination unit that determines that the monitoring target is in an abnormal state, in a case where the divergence degree is greater than or equal to a predetermined level,

wherein the biological information measurement unit measures a heart rate of the monitoring target, as the biological information, and
wherein the abnormality determination unit determines that the monitoring target is in the abnormal state due to paroxysmal atrial fibrillation, in the case where the divergence degree is greater than or equal to the predetermined level.

6. A biological monitoring device comprising:

a biological information measurement unit that measures biological information of a monitoring target for each predetermined cycle;
a biological information holding unit that holds time series data of the biological information measured by the biological information measurement unit;
a dispersion degree calculation unit that calculates a dispersion degree of the biological information in a predetermined measurement period, with reference to the time series data held in the biological information holding unit;
a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and
an abnormality determination unit that determines that the monitoring target is in an abnormal state, in a case where the divergence degree is greater than or equal to a predetermined level,

wherein the biological information measurement unit measures a respiration rate of the monitoring target, as the biological information, and
wherein the abnormality determination unit determines that the monitoring target is in the abnormal state due to Cheyne-Stokes respiration, in the case where the divergence degree is greater than or equal to the predetermined level.

7. A biological monitoring device comprising:

a biological information measurement unit that measures biological information of a monitoring target for each predetermined cycle;

a biological information holding unit that holds time series data of the biological information measured by the biological information measurement unit;

a dispersion degree calculation unit that calculates a dispersion degree of the biological information in a predetermined measurement period, with reference to the time series data held in the biological information holding unit;

a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and

an abnormality determination unit that determines that the monitoring target is in an abnormal state, in a case where the divergence degree is greater than or equal to a predetermined level,

wherein the biological information measurement unit measures a respiration rate and a posture of the monitoring target, as the biological information, and

wherein the abnormality determination unit determines that the monitoring target is in the abnormal state due to orthopnea, in the case where the divergence degree relating to the respiration rate is greater than or equal to the predetermined level and a posture change of the monitoring target is detected from measurement data of the posture.

8. The biological monitoring device according to claim 1, wherein the monitoring target is a living organism lying on a bed,

wherein the biological information measurement unit measures a movement of the monitoring target, as the biological information, and

wherein the abnormality determination unit determines that the monitoring target is in the abnormal state of having a high possibility of falling off the bed, in the case where the divergence degree is greater than or equal to the predetermined level.

9. A biological monitoring program causing a computer to function as:

a biological information measurement unit that measures biological information of a monitoring target for each predetermined cycle;

a biological information holding unit that holds time series data of the biological information measured by the biological information measurement unit;

a dispersion degree calculation unit that calculates a dispersion degree of the biological information in a predetermined measurement period, with reference to the time series data held in the biological information holding unit;

a divergence degree calculation unit that calculates, for evaluation biological information which is the biological information measured by the biological information measurement unit at a predetermined time point after the measurement period, a divergence degree of the evaluation biological information at the predetermined time point from a dispersion tendency of past biological information in the measurement period, based on the dispersion degree; and

an abnormality determination unit that determines that the monitoring target is in an abnormal state, in a case where the divergence degree is greater than or equal to a predetermined level,

wherein the biological information measurement unit measures a plurality of types of biological information,

wherein the biological information holding unit holds time series data of the plurality of types of biological information measured by the biological information measurement unit,

wherein the dispersion degree calculation unit individually calculates a dispersion degree of each of the plurality of types of biological information in the measurement period, with reference to the time series data of the plurality of types of biological information held in the biological information holding unit, and

wherein the divergence degree calculation unit: calculates, for the evaluation biological information of each of the plurality of types at the predetermined time point, a divergence degree of the evaluation biological information from a dispersion tendency of the biological information in the measurement period, based on the dispersion degree; and calculates a weighted average of the calculated divergence degrees, as the divergence degree that is compared with the predetermined level by the abnormality determination unit.

**Statement under Art. 19.1 PCT**

Claims 1 and 9 were amended to limit the biological information measurement unit, the biological information holding unit, the variation degree calculation unit, and the divergence degree calculation unit. These amendments are based on claim 4 and paragraphs [0029], [0030], [0035], [0036], [0045] to [0058], and [0062] to [0070] of the specification as originally filed.

Claims 5 to 7 were amended to be in independent form. These amendments are based on claims 1 and 5 to 7 as originally filed.

## FIG.1

FIG.2

## FIG.3A

MEDCARE Hospital 3F

| | | |
|---|---|---|
| 301-1 TARO AKASHI | 303-1 KAZUO YUHASHI | 305 TARO YOSHIDA |
| 301-2 JIRO KATO | 303-2 YOSHIO KATO | |
| 301-3 SABURO KISHIKAWA | 303-3 MASAO SAITO | 306 JIRO MIYOSHI |
| 301-4 SHIRO HISANO | 303-4 TAKAO SUDO | 307 SABURO SAKUMA |
| 302-1 RYOKO TANAKA | 304-1 RYOKO AKIBA | 308 SHIRO KISHIMOTO |
| 302-2 MASAKO OGASAWARA | 304-2 MASAKO SASAKI | |
| 302-3 KYOKO NAKAYAMA | 304-3 KYOKO MIYAGAWA | MEN'S RESTROOM |
| 302-4 KEIKO YOSHIKAWA | 304-4 KEIKO SAITO | LADIES' RESTROOM |

Vital Monitoring
Layout Change
Setting

## FIG.3B

MEDCARE Hospital 3F

301-1 TARO AKASHI
301-2 JIRO KATO
301-3 SAB
301-4 SHI
302-1 RYO
302-2 MA
302-3 KYO
302-4 KEI

303-1 KAZUO YUHASHI
303-2 YOSHIO KATO

305 TARO YOSHIDA

Calling···

No.1   301-1 TARO AKASHI (RISK OF FALLING)

No.2   302-3 KYOKO NAKAYAMA (PULSE ABNORMALITY)

OK

Vital Monitoring
Layout Change
Setting

FIG.4

MEDCARE Hospital 3F

# Calling · · ·

No.1 301-1 TARO AKASHI (RISK OF FALLING)

No.2 302-3 KYOKO NAKAMURA
        (PULSE ABNORMALITY)

(S) System    CALL FROM MR. TARO AKASHI IN 301-1.
THIS IS "ACTIVE CALL",
AND URGENCY DEGREE IS "HIGH".
                                    15:36

(S) System    CALL FROM MS. KYOKO NAKAMURA IN
301-2. URGENCY DEGREE IS "MEDIUM".
                                    15:37

## FIG.5

START

STEP1
$1 \rightarrow ct$

STEP2
RECEIVE MEASUREMENT DATA (SDAT1, SDAT2, ..., SDATn)
OF EACH VITAL SENSOR FROM NURSE CALL BASE UNIT

STEP3
$MD1[ct] \leftarrow SDAT1, \quad MD2[ct] \leftarrow SDAT2,$
$..., \quad MDn[ct] \leftarrow SDATn$

STEP4
$ct \leftarrow ct+1$

STEP5
NO $\quad ct = m+1 ?$

YES

B

STEP6
CALCULATE STANDARD DEVIATIONS
$\sigma 1$ TO $\sigma n$ FOR MD1 TO MDn

STEP7
RECEIVE MEASUREMENT DATA (SDAT1, SDAT2, ..., SDATn)
OF EACH VITAL SENSOR FROM NURSE CALL BASE UNIT

STEP8
$MD1[m+1] \leftarrow SDAT1, \quad MD2[m+1] \leftarrow SDAT2,$
$..., \quad MDn[m+1] \leftarrow SDATn$

A

# FIG.6

```
         ╱A╲

                                                STEP9
┌──────────────────────────────────────────────┐
│  CALCULATE DEVIATION VALUES c1 TO cn BASED ON  │
│  σ1 TO σn AND MD1[m + 1] TO MDn[m + 1]         │
└──────────────────────────────────────────────┘

                                                STEP10
┌──────────────────────────────────────────────┐
│  CALCULATE URGENCY DEGREE SCORE (EmS)          │
│  BASED ON DEVIATION VALUES c1 TO cn            │
└──────────────────────────────────────────────┘

                                      STEP11
        ╱────────────────────────╲
       ╱  EmS ≦ Emth2 or           ╲    YES
       ╲  Emth1 ≦ EmS ?            ╱──────────┐
        ╲────────────────────────╱           │
                    NO                        │
                                    STEP20    │
                  STEP12       ┌──────────────────────────────┐
          ┌──────────────┐     │  ABNORMALITY                 │
          │  ct ← 1      │     │  NOTIFICATION PROCESS        │
          └──────────────┘     └──────────────────────────────┘

                                      STEP13
  ┌────────────────────────────────────────────┐
  │  MD1[ct] ← MD1[ct+1], MD2[ct] ← MD2[ct+1],  │
  │  ..., MDn[ct] ← MDn[ct+1]                    │
  └────────────────────────────────────────────┘

                                      STEP14
              ┌──────────────┐
              │  ct ← ct+1   │
              └──────────────┘

                                      STEP15
        ╱────────────────────────╲
  NO   ╱       ct = m+1 ?          ╲
───────╲                          ╱
        ╲────────────────────────╱
                    YES

                  ╲B╱
```

FIG.7

## INTERNATIONAL SEARCH REPORT

| International application No. |
| --- |
| PCT/JP2016/057832 |

| A. CLASSIFICATION OF SUBJECT MATTER |
| --- |
| ***A61B5/00***(2006.01)i |

According to International Patent Classification (IPC) or to both national classification and IPC

| B. FIELDS SEARCHED |
| --- |
| Minimum documentation searched (classification system followed by classification symbols)<br>A61B5/00-5/053, A61B5/06-5/22 |

| Documentation searched other than minimum documentation to the extent that such documents are included in the fields searched |
| --- |
| Jitsuyo Shinan Koho          1922-1996   Jitsuyo Shinan Toroku Koho   1996-2016<br>Kokai Jitsuyo Shinan Koho    1971-2016   Toroku Jitsuyo Shinan Koho   1994-2016 |

| Electronic data base consulted during the international search (name of data base and, where practicable, search terms used) |
| --- |

### C. DOCUMENTS CONSIDERED TO BE RELEVANT

| Category* | Citation of document, with indication, where appropriate, of the relevant passages | Relevant to claim No. |
| --- | --- | --- |
| Y<br>A | Toshikazu WADA et al., "Anomaly Monitoring of Time Series Data based on Gaussian Process Regression", The Transactions of the Institute of Electronics, Information and Communication Engineers, 01 December 2013 (01.12.2013), vol.J96-D, no.12, pages 3068 to 3078 | 1-3,8,9<br>4-7 |
| Y | JP 2003-290174 A (Softrox Co., Ltd.), 14 October 2003 (14.10.2003), paragraphs [0009], [0010] (Family: none) | 1-3,8,9 |
| Y | JP 2010-108368 A (Carecom Co., Ltd.), 13 May 2010 (13.05.2010), paragraph [0004] (Family: none) | 8 |

☐ Further documents are listed in the continuation of Box C.      ☐ See patent family annex.

| * Special categories of cited documents: | |
| --- | --- |
| "A" document defining the general state of the art which is not considered    to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 25 May 2016 (25.05.16) | 07 June 2016 (07.06.16) |

| Name and mailing address of the ISA/ | Authorized officer |
| --- | --- |
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2012239799 A